# EUROPÄISCHE PATENTSCHRIFT

(11) **EP 0 413 692 B1**
(45) Veröffentlichungstag und Bekanntmachung des Hinweises auf die Patenterteilung: **20.10.1993**
(21) Anmeldenummer: 89902638.9
(22) Anmeldetag: 02.02.1989
(51) Int. Cl.: C07C 403/00, C07D 257/04, A61K 31/07, A61K 7/42

(54) **VERWENDUNG VON HEXATRIENDERIVATEN ZUR HERSTELLUNG VON MITTELN GEGEN AKNE, PSORIASIS UND LICHTSCHÄDEN DER HAUT**
USE OF HEXATRIENE DERIVATIVES FOR THE MANUFACTURE OF PREPARATIONS FOR TREATING ACNE, PSORIASIS AND LIGHT-INDUCED DAMAGE TO THE SKIN
EMPLOI DE DERIVES D'HEXATRIENE POUR LA FABRICATION DE PREPARATIONS PERMETTANT DE TRAITER L'ACNE, LE PSORIASIS ET LES PHOTOTRAUMATISMES DE LA PEAU

(43) Veröffentlichungstag der Anmeldung: 27.02.1991
(73) Patentinhaber: BASF Aktiengesellschaft, 67063 Ludwigshafen (DE)
(72) Erfinder: WÜST, Hans-Heiner, D-6901 Dossenheim (DE); FRICKEL, Fritz-Frieder, D-6705 Deidesheim (DE); PAUST, Joachim, D-6701 Neuhofen (DE); SCHMIEDER, Klaus, D-2948 Schortens 1 (DE); NÜRRENBACH, Axel, D-6718 Grünstadt 1 (DE)
(86) Internationale Anmeldenummer: EP8900104
(87) Internationale Veröffentlichungsnummer: WO9008760

(56) Entgegenhaltungen:
- EP-A- 0 253 393
- J. MED. CHEM., Band 27, Nr. 11, 1984, M. I. DAWSON et al: "Conformationally restricted retinoids", Seite 1516-1531
- J. MED. CHEM., Band 24, Nr. 5, 1981, M. I. DAWSON et al: "Aromatic retinoic acid analogues. Synthesis and pharmacological activity", Seite 583-592
- J. MED. CHEM., Band 23, Nr. 9, 1980, M. I. DAWSON et al: "Retinoic acid analogues. Synthesis and potential as cancer chemopreventive agents", Seite 1013-1022
- CHEMICAL ABSTRACTS, Band 99, Nr. 11, 12. September 1983, Columbus, Ohio, USA; M. I. DAWSON et al: "Aromatic retinoic acid analogs. 2. Synthesis and pharmacological activity", Seite 569, Zusammenfassung Nr. 88414r

## Beschreibung

Die Erfindung betrifft die Verwendung von Hexatrienderivaten bei der Herstellung von therapeutischen und kosmetischen Mitteln gegen Vitiligo, trockene Augen sowie rheumatische und arthritische Erkrankungen.

Es ist bekannt, daß Polyenverbindungen wie Retinol oder Retinsäure, und auch solche Verbindungen, bei denen ein oder mehrere aromatische Ringe in die Polyenstruktur inkorporiert sind, pharmakologische Wirkungen bei der topischen und systemischen Therapie von Neoplasien und Dermatosen, z.B. Akne oder Psoriasis, aufweisen (R.C. Moon and L.M. Hri in "The Retinoids" (Ed. M.B. Sporn, A.B. Roberts and D.S. Goodman), Vol. 2, p 327 ff, Academic Press, Inc. 1984; G.L. Peck, ibid. Vol. 2, p. 391 ff). Sie haben jedoch auch unerwünschte Nebenwirkungen.

Aus M.I. Dawson et al., J. Med. Chem. 27 (1984), 1516 bis 1531 ist bekannt, daß 4-[(E)-3-Methyl-6-(2,6,6-Trimethylcyclohexen-1-yl)1,3,5-hexatrienyl]benzoesäure bei je einem in-vivo- und in-vitro-Test-Modell anticancerogene Wirkung Zeigt.

Der Erfindung lag die Aufgabe zugrunde, Mittel gegen Vitiligo, trockene Augen sowie rheumatische und arthritische Erkrankungen zu entwickeln.

Es wurde nun gefunden, daß substituierte Hexatrienderivate der Formel I 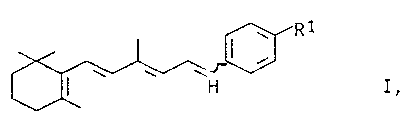
in der R¹ eine Methylgruppe, eine Nitril- oder C₂- bis C₁₀-Ketalgruppe der Formel 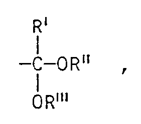
wobei R', R'' und R''' Alkylgruppen mit 1 bis 9 C-Atomen darstellen, die Summe der C-Atome von R', R'' und R''' 2 bis 10 beträgt und R' auch Wasserstoff sein kann, einen Oxazolinyl- oder Tetrazolylrest, oder die Reste -CH₂OR², -CH₂NR³R⁴ oder -COR⁵ bedeutet, worin
- R²: ein Wasserstoffatom, eine C₁-₂₀-Alkanoyl- oder eine gegebenenfalls substituierte Benzoylgruppe,
- R³ und R⁴: Wasserstoffatome, eine C₁-₄-Alkyl-, C₁-₆-Alkanoyl- oder eine gegebenenfalls substituierte Benzoylgruppe oder zusammen mit dem Stickstoffatom, an das sie gebunden sind, einen heterocyclischen Rest, und
- R⁵: ein Wasserstoffatom, eine C₁-₄-Alkylgruppe, ein Halogenatom oder die Reste -OR⁶ oder -NR⁷R⁸ bedeuten, wobei
- R⁶: für ein Wasserstoffatom, eine gegebenenfalls durch Hydroxylgruppen substituierte C₁-C₆-Alkylgruppe oder eine gegebenenfalls substituierte Aryl- oder gegebenenfalls im Arylteil substituierte Aralkylgruppe, und
- R⁷ und R⁸: für Wasserstoffatome, gegebenenfalls durch Hydroxylgruppen substituierte C₁-C₆-Alkylgruppen oder gegebenenfalls substituierte Aryl- oder gegebenenfalls im Arylteil substituierte Aralkylgruppen oder zusammen mit dem Stickstoffatom, an das sie gebunden sind, für einen heterocyclischen Rest stehen,
sowie gegebenenfalls deren physiologisch verträgliche Salze eine verbesserte Wirkung gegen Vitiligo, trockene Augen sowie rheumatische und arthritische Erkrankungen, vor allem bezüglich der therapeutischen Breite, besitzen.

R¹ ist bevorzugt eine COOH-Gruppe.

Als Arylgruppe ist für R⁶, R⁷ und R⁸ die Phenylgruppe bevorzugt, die mit einer Methyl-, Methoxy- oder Nitrogruppe substituiert sein kann; als Aralkylgruppe ist für R⁶, R⁷ und R⁸ die Benzylgruppe bevorzugt, die im Arylteil mit einer Methyl- oder Methoxygruppe oder mit Halogen substituiert sein kann. Substituenten der Benzoylgruppe können beispielsweise die Methyl- oder die Methoxygruppe oder Halogen sein. Als heterocyclische Reste für R³ und R⁴ bzw. R⁷ und R⁸ kommen vorzugsweise der Pyrrolidino-, Piperidino- oder Morpholinorest in Betracht. Als Halogenatome sind für R⁵ vorzugsweise Fluor und Chlor zu nennen.

Die 1E-Verbindungen sind gegenüber den 1Z-Verbindungen bevorzugt.

Typische Beispiele für erfindungsgemäß zu verwendende Verbindungen sind:
(all-E)-1-(4-Carboxyphenyl)-4-methyl-6-(2,6,6-trimethyl-1-cyclohexen-1-yl)-1,3,5-hexatrien
(all-E)-1-(4-Carbomethoxyphenyl)-4-methyl-6-(2,6,6-trimethyl-1-cyclohexen-1-yl)-1,3,5-hexatrien
(all-E)-1-(4-Carbethoxyphenyl)-4-methyl-6-(2,6,6-trimethy-1-cyclohexen-1-yl)-1,3,5-hexatrien
(all-E)-1-(4-Carbopropyloxphenyl)-4-methyl-6-(2,6,6-trimethyl-1-cyclohexen-1-yl)-1,3,5-hexatrien
(all-E)-1-(4-Carbobutoxyphenyl)-4-methyl-6-(2,6,6-trimethyl-1-cyclohexen-1-yl)-1,3,5-hexatrien
(all-E)-1-(4-Cyanophenyl)-4-methyl-6-(2,6,6-trimethyl-1-cyclohexen-1-yl)-1,3,5-hexatrien
(all-E)-1-(4-Fluorcarbonylphenyl)-4-methyl-6-(2,6,6-trimethyl-1-cyclohexen-1-yl)-1,3,5-hexatrien
(all-E)-1-(4-Chlorcarbonylphenyl)-4-methyl-6-(2,6,6-trimethyl-1-cyclohexen-1-yl)-1,3,5-hexatrien
(all-E)-1-(4-Carbamoylphenyl)-4-methyl-6-(2,6,6-trimethyl-1-cyclohexen-1-yl)-1,3,5-hexatrien
(all-E)-1-[4-(Butylcarbamoyl)phenyl]-4-methyl-6-(2,6,6-trimethyl-1-cyclohexen-1-yl)-1,3,5-hexatrien
(all-E)-1-[4-(Diethylcarbamoyl)phenyl]-4-methyl-6-(2,6,6-trimethyl-1-cyclohexen-1-yl)-1,3,5-hexatrien
(all-E)-1-[4-(2-Hydroxyethylcarbamoyl)phenyl]-4-methyl-6-(2,6,6-trimethyl-1-cyclohexen-1-yl)-1,3,5-hexatrien
(all-E)-1-[4-(Phenylcarbamoyl)phenyl]-4-methyl-6-(2,6,6-trimethyl-1-cyclohexen-1-yl)-1,3,5-hexatrien
(all-E)-1-[4-(Benzylcarbamoyl)phenyl)]-4-methyl-6-(2,6,6-trimethyl-1-cyclohexen-1-yl)-1,3,5-hexatrien
(all-E)-1-(4-Formylphenyl)-4-methyl-6-(2,6,6-trimethyl-1-cyclohexen-1-yl)-1,3,5-hexatrien
(all-E)-1-(4-Acetylphenyl)-4-methyl-6-(2,6,6-trimethyl-1-cyclohexen-1-yl)-1,3,5-hexatrien
(all-E)-1-[4-(Hydroxymethyl)phenyl]-4-methyl-6-(2,6,6-trimethyl-1-cyclohexen-1-yl)-1,3,5-hexatrien
(all-E)-1-[4-(Methoxymethyl)phenyl]-4-methyl-6-(2,6,6-trimethyl-1-cyclohexen-1-yl)-1,3,5-hexatrien
(all-E)-1-[4-(Phenoxymethyl)phenyl]-4-methyl-6-(2,6,6-trimethyl-1-cyclohexen-1-yl)-1,3,5-hexatrien
(all-E)-1-[4-(Benzyloxymethyl)phenyl]-4-methyl-6-(2,6,6-trimethyl-1-cyclohexen-1-yl)-1,3,5-hexatrien
(all-E)-1-[4-(Formyloxymethyl)phenyl]-4-methyl-6-(2,6,6-trimethyl-1-cyclohexen-1-yl)-1,3,5-hexatrien
(all-E)-1-[4-(Acetoxymethyl)phenyl]-4-methyl-6-(2,6,6-trimethyl-1-cyclohexen-1-yl)-1,3,5-hexatrien
(all-E)-1-[4-(Propionyloxymethyl)phenyl]-4-methyl-6-(2,6,6-trimethyl-1-cyclohexen-1-yl)-1,3,5-hexatrien
(all-E)-1-[4-(Hexadecanoyloxymethyl)phenyl]-4-methyl-6-(2,6,6-trimethyl-1-cyclohexen-1-yl)-1,3,5-hexatrien
(all-E)-1-[4-(Aminomethyl)phenyl]-4-methyl-6-(2,6,6-trimethyl-1-cyclo-hexen-1-yl)-1,3,5-hexatrien
(all-E)-1-[4-(N,N-Diethylaminomethyl)phenyl]-4-methyl-6-(2,6,6-trimethyl-1-cyclohexen-1-yl)-1,3,5-hexatrien
(all-E)-1-[4-(pyrrolidinomethyl)phenyl]-4-methyl-6-(2,6,6-trimethyl-1-cyclohexen-1-yl)-1,3,5-hexatrien
(all-E)-1-[4-(Piperidinomethyl)phenyl]-4-methyl-6-(2,6,6-trimethyl-1-cyclohexen-1-yl)-1,3,5-hexatrien
(all-E)-1-[4-(N-Formylaminomethyl)phenyl]-4-methyl-6-(2,6,6-trimethyl-1-cyclohexen-1-yl)-1,3,5-hexatrien
(all-E)-1-[4-(N-Acetylaminomethyl)phenyl]-2,6,6-trimethyl-1-cyclohexen-1-yl)-1,3,5-hexatrien
(all-E)-1-[4-(N-Benzoylaminomethyl)phenyl]-4-methyl-6-(2,6,6-trimethyl-1 -cyclohexen-1-yl)-1,3,5-hexatrien
(all-E)-1-[4-(Dioxolan-2-yl)phenyl]-4-methyl-6-(2,6,6-trimethyl-1-cyclohexen-1-yl)-1,3,5-hexatrien
(all-E)-1-[4-(Oxazolin-2-yl)phenyl]-4-methyl-6-(2,6,6-trimethyl-1-cyclohexen-1-yl)-1,3,5-hexatrien
(all-E)-1-[4-(Tetrazol-5-yl)phenyl]-4-methyl-6-(2,6,6-trimethyl-1-cyclohexen-1-yl)-1,3,5-hexatrien
(1Z,3E,5E)-1-(4-Carboxyphenyl)-4-methyl-6-(2,6,6-trimethyl-1-cyclohexen-1-yl)-1,3,5-hexatrien
(1Z,3E,5E)-1-(4-Carbomethoxyphenyl)-4-methyl-6-(2,6,6-trimethyl-1-cyclohexen-1-yl)-1,3,5-hexatrien
(1Z,3E,5E)-1-(4-Carbethoxyphenyl)-4-methyl-6-(2,6,6-trimethyl-1-cyclohexen-1-yl)-1,3,5-hexatrien
(1Z,3E,5E)-1-(4-Cyanophenyl)-4-methyl-6-(2,6,6-trimethyl-1-cyclohexen-1-yl)-1,3,5-hexatrien
Die erfindungsgemäß zu verwendenden Verbindungen kann man herstellen, indem man ein Phosphoniumsalz der Formel II 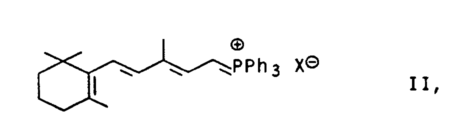
worin X^{⊖} für Anionen wie Chlorid, Bromid oder vorzugsweise Hydrogensulfat steht, mit einem aromatischen Aldehyd der Formel III 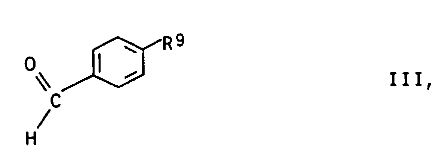
wobei R⁹ die Methyl- oder Nitrilgruppe oder eine Gruppe -COOR¹⁰ und R¹⁰ ein Wasserstoffatom oder eine C₁-₃-Alkylgruppe bedeutet, in einer Wittig-Reaktion umsetzt. Zweckmäßigerweise arbeitet man in einem Lösungsmittel in Gegenwart der für Wittig-Reaktionen üblichen basischen Verbindungen.

Die Umsetzung nach Wittig verläuft bei einer Temperatur von bis zu 100°C, zweckmäßig bei 20 bis 50°C. Die Umsetzung kann bei atmosphärischem Druck oder in einem geschlossenen Gefäß unter erhöhtem Druck, gegebenenfalls unter Erwärmung auf den angegebenen Temperaturbereich durchgeführt werden.

Man kann diese Umsetzung in Gegenwart eines Verdünnungs- oder Lösungsmittels, beispielsweise eines niederen gesättigten Dialkylethers, Dialkylglykolethers oder cyclischen Ethers, wie Diethylether, Ethyl-tert.-butylether, 1,2-Dimethoxyethan, Tetrahydrofuran oder Dioxan, eines aromatischen Kohlenwasserstoffs, wie Benzol oder eines Alkylbenzols, wie Toluol oder Xylol, oder eines gesättigten aliphatischen Kohlenwasserstoffs, wie Hexan, Heptan oder Isooctan, eines niederen aliphatischen Ketons, wie Aceton, Methylethylketon oder Methylisobutylketon, eines Dialkylformamids, wie Dimethyl- oder Diethylformamid, oder in Mischungen der genannten Lösungsmittel durchführen. Bevorzugt verwendet man cyclische Ether, wie Dioxan oder Tetrahydrofuran sowie insbesondere Dimethylformamid oder deren Mischungen, wobei die Umsetzung im allgemeinen bei einer Temperatur bis zu 30°C abläuft.

Die Umsetzungen werden in Gegenwart eines Deprotonierungsmittels für das Phosphoniumsalz II vorgenommen. Geeignet sind Alkalimetallhydride und Alkalimetallamide, insbesondere des Natriums und Kaliums, die Natrium- oder Kaliumsalze von Dimethylsulfoxid, Alkyllithiumverbindungen wie n-Butyllithium oder Alkalimetallalkoholat, vorzugsweise Natriummethanolat und Natriumethanolat.

Man kann die erfindungsgemäß zu verwendenden Verbindungen auch durch die Wittig-Horner-Variante erhalten, wobei der Aldehyd IV 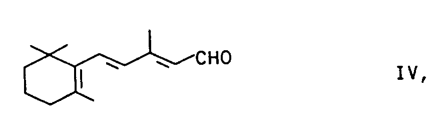
mit einem Phosphonat der Formel V 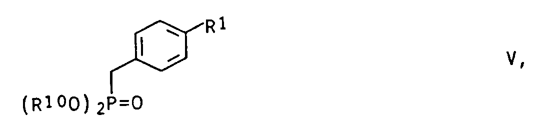
in der R¹ die oben angegebene Bedeutung hat und R¹⁰ für einen C₁-₄-Alkylrest steht, umgesetzt wird. Diese Umsetzungen verlaufen ähnlich den oben beschriebenen Wittig-Reaktionen in Gegenwart eines geeigneten Deprotonierungsmittels.

Die Wittig- oder Wittig-Horner-Reaktion liefert üblicherweise Gemische der sterisch isomeren (E/Z)-Olefine.

E/Z-Isomerengemische mit überwiegendem Z-Anteil werden unter Lichteinwirkung an der olefinischen Doppelbindung zu Gemischen mit höherem Anteil der (E)-Isomeren umgelagert. Aus den erhaltenen (E/Z)-Isomerengemischen mit jetzt günstigerem (E)-Gehalt werden vorteilhaft reine (E)-Verbindungen der Formel (I), vorzugsweise durch Kristallisation oder eine chromatographische Methode wie die Säulen- oder präparative HPL-Chromatographie erhalten.

Die photoisomerisierung wird vorzugsweise in Lösung durchgeführt. Geeignete Lösungsmittel sind polare protische oder aprotische Lösungsmittel, z.B. Methanol, Ethanol, Essigester, Tetrahydrofuran, Aceton. Die Konzentration der bestrahlten Lösung liegt bei 0,1 bis 50, vorzugsweise bei 1 bis 15 Gewichtsprozent.

Man kann in Gegenwart von Sensibilisatoren wie beispielsweise Acetophenon, 4-Methoxyacetophenon, Propiophenon, Benzol, Aceton, Benzophenon, Benzil, Michlers Keton bestrahlen. Besonders bevorzugt ist hier Aceton.

Als Lichtquelle zur Durchführung der genannten Photoreaktion können künstliche Strahler, deren Emission zumindest teilweise im Bereich von 200 bis 600 nm, vorzugsweise im Bereich von 300 bis 400 nm liegt, verwendet werden. Vorteilhaft sind Quecksilberdampf-, Fluor-, Xenon- oder Wolframlampen, Leuchtstoffröhren oder Kohlebogenlampen.

Die Bestrahlungstemperatur ist abhängig von der Art des verwendeten Lösungsmittels. Besonders bevorzugt ist der Bereich von +10 bis +30°C. Die Strahlungswärme kann durch Lampenkühlung und/oder Kühlung des Reaktionsgemisches abgeführt werden, wobei im Lampenkühlkreis destilliertes Wasser oder in bekannter Weise mit Zusätzen versehene, filternde Lösungen eingesetzt werden können.

Die Benzoesäureester der allgemeinen Formel I werden, falls dies erwünscht ist, in die freien Carbonsäuren und ihre physiologisch verträglichen Salze, durch Esterverseifung überführt. Umgekehrt läßt sich natürlich die freie Säure in bekannter Weise verestern.

Zweckmäßigerweise wird die Verseifung/Veresterung in Gegenwart eines Verdünnungs- oder Lösungsmittels, beispielsweise eines Dialkylglykolethers oder cyclischen Ethers, wie 1,2-Dimethoxyethan, Tetrahydrofuran oder Dioxan, eines niederen aliphatischen Ketons, wie Aceton, Methylethylketon oder Methylisobutylketon, oder in einem niederen aliphatischen Alkohol, wie Methanol, Ethanol, Propanol oder Isopropanol, gegebenenfalls in Gegenwart von Wasser bzw. in Mischungen der genannten Lösungsmittel mit Wasser durchgeführt.

Bevorzugte Lösungsmittel sind wäßrige Mischungen von Ethanol und Methanol, wobei die Umsetzung beim Siedepunkt des Reaktionsgemisches durchgeführt wird.

Die Verseifung geschieht bevorzugt in Gegenwart von Alkali, wie Alkalimetallhydroxide, Alkalimetallcarbonate und -hydrogencarbonate, insbesondere des Natriums und Kaliums, organischen tertiären Basen, wie Pyridin oder niedere Trialkylamine, wie Trimethyl- oder Triethylamin in Gemischen mit Wasser. Dabei wird die verwendete Base im Verhältnis zum Ester in stöchiometrischer Menge oder in geringem Überschuß eingesetzt. Vorzugsweise wird Natrium- oder Kaliumhydroxid verwendet.

Die erfindungsgemäß zu verwendenden Amide können in an sich bekannter Weise hergestellt werden, indem man die entsprechenden Benzoesäuren zunächst in carbonylaktivere Derivate überführt, z.B. in die Säurehalogenide, -azide, -imidazolide, oder -anhydride, die O-Acyl-N,N'-dicyclohexylisoharnstoffe oder p-Nitrophenylester, und diese mit Aminen HNR⁷R⁸ behandelt. In den Fällen besonders reaktiver Amine, vor allem Ammoniak, ist die direkte Amidolyse von Estern (mit dem Rest -OR⁶) bevorzugt.

Ein Halogenid einer Carbonsäure, vorzugsweise das Säurechlorid, kann durch Reaktion mit 2-Aminoethanol und anschließende Cyclisierung in ein Oxazolinderivat der Formel I überführt werden.

Eine Carbonsäure, ein Carbonsäureester oder ein Carbonsäureamid der Formel I kann in an sich bekannter Weise zu den entsprechenden Alkoholen bzw. Aminen reduziert werden. Vorteilhaft wird die Reduktion mit Hilfe eines Metallhydrids oder Alkalimetallhydrids in Gegenwart eines geeigneten Lösungsmittels durchgeführt. Als Metallhydride werden vorzugsweise komplexe Metallhydride wie Lithiumaluminiumhydrid oder Diisobutylaluminiumhydrid eingesetzt. Als Lösungsmittel werden beim Arbeiten mit Lithiumaluminiumhydrid Ether eingesetzt, wie Diethylether, Dioxan oder Tetrahydrofuran. Führt man die Reduktion mit Diisobutylaluminiumhydrid oder einem Alkoxynatriumaluminiumhydrid durch, so ist die Verwendung von Kohlenwasserstoffen wie Hexan oder Toluol bevorzugt.

Ein Amin oder ein Alkohol der Formel I kann in an sich bekannter Weise mit einem Alkanoylhalogenid oder -anhydrid, einem Aralkylhalogenid oder -anhydrid oder einem Aroyl- oder Heteroaroylhalogenid oder -anhydrid zweckmäßigerweise in einem inerten Verdünnungs- oder Lösungsmittel, z.B. einem niederen aliphatischen Keton wie Aceton, Methylethylketon oder Methylisobutylketon, einem Dialkylformamid wie Dimethylformamid oder Diethylformamid oder mit überschüssigem Acylierungsmittel als Verdünnungs- oder Lösungsmittel in die erfindungsgemäßen Amide und Ester überführt werden. Bevorzugt werden die Umsetzungen in Gegenwart einer Base als säurebindendem Mittel in einem zwischen -20°C und dem Siedepunkt des Reaktionsgemisches liegenden Temperaturbereich vorgenommen. Geeignete Basen sind Alkalimetallcarbonate, -hydrogencarbonate, -hydroxide oder -alkoholate, insbesondere des Natriums und Kaliums, basische Oxide, wie Aluminiumoxid oder Calciumoxid, organische tertiäre Basen, wie Pyridin, oder niedere Trialkylamine, wie Trimethyl- oder Triethylamin. Dabei können die Basen im Verhältnis zum eingesetzten Alkylierungsmittel in katalytischer Menge oder in stöchiometrischer Menge bzw. in geringem Überschuß verwendet werden.

Ein Alkohol der Formel I kann mit Alkylhalogeniden R²-J, R²-Br oder R²-Cl in Gegenwart von Alkalimetallhydriden, vorzugsweise Natriumhydrid oder in Gegenwart von Alkyllithium-Verbindungen, vorzugsweise n-Butyllithium, in einem organischen Lösungsmittel wie Tetrahydrofuran, Dioxan, 1,2-Dimethoxyethan, Methyl-tert.-butylether oder bei Verwendung von Natriumhydrid auch in Dimethylformamid in einem zwischen -10°C und 40°C liegenden Temperaturbereich zu einem Ether der Formel I umgesetzt werden.

Ein Alkohol der Formel I kann mit geeigneten Oxidationsmitteln, vorzugsweise Mangan(IV)-oxid, gegebenenfalls auf einem anorganischen Trägermaterial wie Silicagel oder Aluminiumoxid zu einem Aldehyd der Formel I oxidiert werden. Vorteilhaft arbeitet man in einem inerten organischen Lösungsmittel, beispielsweise einem Kohlenwasserstoff wie Hexan oder in einem Ether wie beispielsweise Tetrahydrofuran oder in Mischungen der genannten Lösungs- und Verdünnungsmittel im Temperaturbereich zwischen -10°C und 30°C. Die benötigte Reaktionszeit ist im wesentlichen von der Oxidationsaktivität des eingesetzten Mangan(IV)-oxids abhängig.

Einen Aldehyd der Formel I kann man auch durch Reduktion des entsprechenden Nitrils der Formel I mit Diisobutylaluminiumhydrid in einem Lösungsmittel, vorzugsweise in Toluol, Hexan, Tetrahydrofuran oder Mischungen dieser Lösungsmittel in einem Temperaturbereich zwischen -40°C und Raumtemperatur erhalten.

Ein Nitril der Formel I kann in an sich bekannter Weise unter Säure- oder vorteilhafter Basenkatalyse zur entsprechenden Carbonsäure verseift werden. Als Basen bevorzugt sind Alkalimetallhydroxide, besonders Kaliumhydroxid, das im Überschuß eingesetzt wird. Als Lösungsmittel werden in der Regel mit Wasser mischbare Alkohole wie z.B. Methanol, Ethanol, Isopropanol oder n-Butanol eingesetzt. Die Umsetzung wird üblicherweise beim Siedepunkt des Reaktionsgemisches durchgeführt.

Aus den Nitrilen der Formel I können durch Addition eines Azids, z.B. eines Alkalimetallazids, vorzugsweise Natriumazid, in Gegenwart von Aluminiumchlorid oder Ammoniumchlorid die entsprechenden Tetrazole der Formel I erhalten werden. Als Lösungsmittel verwendet man bevorzugt cyclische Ether, wie Dioxan oder Tetrahydrofuran, sowie insbesondere Dimethylformamid oder deren Mischungen, wobei die Umsetzung im allgemeinen bei einer Temperatur von 60 bis 100°C abläuft.

Einige der erfindungsgemäß zu verwendenden Verbindungen weisen ein acides Wasserstoffatom auf und können daher mit Basen in üblicher Weise in ein physiologisch verträgliches, gut wasserlösliches Salz überführt werden. Geeignete Salze sind beispielsweise Ammonium-, Alkalimetall-, insbesondere des Natriums, Kaliums und Lithiums, Erdalkalimetallsalze, insbesondere des Calciums oder Magnesiums, sowie Salze mit geeigneten organischen Basen, wie mit niederen Alkylaminen, z.B. Methylamin, Ethylamin oder Cyclohexylamin, oder mit substituierten niederen Alkylaminen, insbesondere hydroxy-substituierten Alkylaminen, wie Diethanolamin, Triethanolamin oder Tris-(hydroxymethyl)-aminomethan, sowie mit Piperidin oder Morpholin.

Gegebenenfalls werden die erhaltenen erfindungsgemäß zu verwendenden Amine der Formel (I) nach bekannter Verfahrensweise in das Säureadditionssalz einer physiologisch verträglichen Säure überführt. Als übliche physiologisch verträgliche organische oder anorganische Säure kommen beispielsweise in Betracht Salzsäure, Bromwasserstoffsäure, Phosphorsäure oder Schwefelsäure und als organische Säuren beispielsweise Oxalsäure, Maleinsäure, Fumarsäure, Milchsäure, Weinsäure, Äpfelsäure, Zitronensäure, Salicylsäure, Adipinsäure oder Benzoesäure. Weitere können aus Fortschritte der Arzneimittelforschung Band 10, Seiten 224 bis 225, Birkhäuser-Verlag, Basel und Stuttgart, 1966, entnommen werden.

Die erfindungsgemäß zu verwendenden Verbindungen und ihre physiologisch verträglichen Salze können aufgrund ihrer pharmakologischen Eigenschaften bei der topischen und systemischen Prophylaxe und Therapie von Vitiligo, trockenen Augen sowie rheumatischen und arthritischen Erkrankungen dienen.

Dementsprechend sind Gegenstand der Erfindung therapeutische und kosmetische Mittel zur topischen und systemischen Anwendung, die eine Verbindung der Formel (I) neben üblichen Trägerstoffen oder Verdünnungsmitteln als Wirkstoff enthalten, und die Verwendung einer Verbindung der Formel (I) zur Herstellung eines derartigen Arzneimittels oder Kosmetikums gegen Vitiligo, trockene Augen sowie rheumatische und arthritische Erkrankungen.

Die Herstellung der therapeutischen und kosmetischen Mittel oder Zubereitungen erfolgt mit den üblichen flüssigen oder festen Trägerstoffen oder Verdünnungsmitteln und den in üblicher Weise verwendeten pharmazeutisch-technischen oder kosmetischen Hilfsstoffen, entsprechend der gewünschten Applikationsart und mit einer zur Anwendung geeigneten Dosierung, in üblicher Weise, beispielsweise durch Vermischen des Wirkstoffs mit den an sich in solchen Präparaten üblichen festen oder flüssigen Träger- und Hilfsstoffen.

Die Mittel können dementsprechend peroral, parenteral oder topisch verabreicht werden. Derartige Zubereitungen sind beispielsweise Tabletten, Filmtabletten, Dragees, Kapseln, Pillen, Pulver, Lösungen oder Suspensionen, Infusions- oder Injektionslösungen sowie Pasten, Salben, Gele, Cremes, Lotionen, Puder, Lösungen oder Emulsionen und Sprays. Bevorzugt wird die topische Anwendung.

Die therapeutischen und kosmetischen Mittel können die erfindungsgemäß zu verwendenden Verbindungen bei lokaler Anwendung in 0,001 bis 1%iger Konzentration, bevorzugt in 0,01 bis 0,1%iger Konzentration und bei systemischer Anwendung vorzugsweise in einer Einzeldosis von 0,1 bis 50 mg, enthalten und täglich in einer oder mehreren Dosierungen je nach Art und Schwere der Erkrankungen verabreicht werden.

Üblicherweise verwendete pharmazeutisch-technische und kosmetische Hilfsstoffe sind beispielsweise für die lokale Anwendung Alkohole, wie Isopropanol, oxethyliertes Ricinusöl oder oxethyliertes hydriertes Ricinusöl, Polyacrylsäure, Glycerinmonostearat, Paraffinöl, Vaseline, Wollfett, Polyethylenglykol 400, Polyethylenglykol 400-Stearat sowie ethoxylierter Fettalkohol, für die systemische Anwendung Milchzucker, Propylenglykol und Ethanol, Stärke, Talk, Polyvinylpyrrolidon. Den Präparaten kann gegebenenfalls ein Antioxidationsmittel, beispielsweise Tocopherol sowie butyliertes Hydroxyanisol oder butyliertes Hydroxytoluol oder geschmacksverbessernde Zusätze, Stabilisierungsmittel, Emulgiermittel, Gleitmittel usw. zugesetzt werden. Voraussetzung ist, daß alle bei der Herstellung pharmazeutischer und kosmetischer Zubereitungen verwendeten Stoffe toxikologisch unbedenklich sind und mit den verwendeten Wirkstoffen verträglich sind.

Herstellung der erfindungsgemäß zu verwendenden Verbindungen:

### Beispiel 1 (all-E)-1-(4-Cyanophenyl)-4-methyl-6-(2,6,6-trimethyl-1-cyclohexen-1-yl)-1,3,5-hexatrien

Zu einer Lösung von 116,8 g (0,2 mol) β-Ionylidenethyltriphenylphosphonium-hydrogensulfat und 32,7 g (0,25 mol) p-Cyanobenzaldehyd in 0,5 l Methanol wurde bei 0 bis 5°C innerhalb 15 min eine Lösung von 7,8 g (0,34 mol) Natrium in 62 ml Methanol zugetropft. Man ließ 18 h bei Raumtemperatur nachrühren, versetzte dann mit 100 ml Wasser und filtrierte unlösliche Bestandteile ab. Das Filtrat wurde fünfmal mit je 300 ml Heptan/Ether (5:1) extrahiert. Nach Trocknen (Na₂SO₄) und Einengen der organischen Phase blieben 521 g Feststoff zurück. Dieser wurde aus 200 ml Methanol + 100 ml Ethanol und danach nochmals aus 300 ml Heptan umkristallisiert. Man erhielt so 15,1 g Kristallisat (E/Z = 3:1). Durchmaliges Umkristallisieren aus 75 ml Heptan wurden 10,2 g (16 %) der Titelverbindung isomerenrein gewonnen, Schmp. 120 bis 121°C.

### Beispiel 2 (all-E)- und (1Z,3E,5E)-1-(4-Carbethoxyphenyl)-4-methyl-6-(2,6,6-trimethyl-1-cyclohexen-1-yl)-1,3,5-hexatrien

Zu einer Lösung von 95,5 g (0,17 mol)
β-Ionylidenethyltriphenylphosphonium-hydrogensulfat und 37,4 g (0,21 mol) p-Formylbenzoesäureethylester in 400 ml absolutem Ethanol wurde bei 5 bis 10°C innerhalb 25 min eine Lösung von 6,3 g (0,275 mol) Natrium in 135 ml absolutem Ethanol zugetropft. Man ließ 18 h bei Raumtemperatur nachrühren, und engte dann das Reaktionsgemisch auf ein Viertel seines Volumens ein. Der Rückstand wurde mit 250 ml Heptan versetzt und gründlich durchmischt. Dann wurde die Heptanphase abdekantiert und der gesamte Vorgang noch zweimal wiederholt. Die vereinigten Heptanphasen wurden dreimal mit je 250 ml Methanol/Wasser (3:2) gewaschen, getrocknet (Na₂SO₄) und eingeengt. Der Rückstand wurde mittels präparativer HPLC (Kieselgel 60, Heptan + 1 % Essigester) aufgetrennt. Die erste Fraktion enthielt 12,8 g (21 %) 1Z-Isomeres der Titelverbindung als Öl. Die zweite Fraktion bestand aus 13,8 g Kristallisat, das mit 10 ml Ethanol verrührt und abgesaugt wurde. So wurden 3,4 g (11 %) all-E-Isomeres der Titelverbindung, Schmp. 140 bis 141°C, erhalten.

### Beispiel 3 (all-E)-1-(4-Carboxyphenyl)-4-methyl-6-(2,6,6-trimethyl-1-cyclohexen-1-yl)-1,3,5-hexatrien

a) 4 g (12,6 mmol)
   (all-E)-1-(4-Cyanophenyl)-4-methyl-6-(2,6,6-trimethyl-1-cyclohexen-1-yl)-1,3,5-hexatrien aus Beispiel 1 wurden in 60 ml Ethanol + 40 ml 10 N Natronlauge 2 h unter Rückfluß erhitzt. Man goß auf Wasser und säuerte mit konzentrierter Salzsäure an. Der Niederschlag wurde abgesaugt, mit Wasser neutral gewaschen, mit wenig Methanol nachgewaschen und getrocknet. Man erhielt 4 g (94 %) leicht verunreinigtes Produkt vom Schmp. 215°C.
   Zur weiteren Reinigung kristallisierte man einmal aus Toluol/Heptan und ein zweites Mal aus Isopropanol um. Man erhielt 2,6 g (61 %) reine Titelverbindung, Schmp. 218°C.
b) 2,9 g (8 mmol)
   (all-E)-1-(4-Carbethoxyphenyl)-4-methyl-6-(2,6,6-trimethyl-1-cyclohexen-1-yl)-1,3,5-hexatrien aus Beispiel 2 wurden 2,5 h in einer Lösung von 1,1 g Kaliumhydroxid (85%ig) in 35 ml Ethanol unter Rückfluß erhitzt. Nach dem Abkühlen goß man auf 0,5 l Wasser und 50 ml Ethanol, säuerte an, rührte wenige min und saugte den gebildeten Niederschlag ab, welcher gründlich mit Wasser und mehrmals mit wenig Ethanol nachgewaschen wurde. Nach Trocknen blieben 2,6 g (97 %) der Titelverbindung als gelbe Kristalle, Schmp. 219 bis 220°C, zurück.

### Beispiel 4 (1Z,3E,5E)-1-(4-Carboxyphenyl)-4-methyl-6-(2,6,6-trimethyl-1-cyclohexen-1-yl)-1,3,5-hexatrien

Zu einer Lösung aus 112,4 g (0,2 mol) β-Ionylidenethyltriphenylphosphonium-hydrogensulfat und 37,5 g (0,25 mol) p-Carboxybenzaldehyd in 500 ml absolutem Ethanol wurde bei 15°C innerhalb 15 min eine Lösung von 15,6 g (0,68 mol) Natrium in 400 ml absolutem Ethanol zugetropft. Man ließ 18 h bei Raumtemperatur nachrühren, goß danach auf 1 l Wasser, säuerte an und extrahierte 3 mol mit je 250 ml Ether. Der nach Trocknen (Na₂SO₄) und Einengen der vereinigten Etherphasen verbliebene schmierige Rückstand wurde mit 400 ml Ethanol verrieben. Die Kristalle wurden abgesaugt und mehrmals mit insgesamt 200 ml Aceton gewaschen. Umkristallisieren aus Essigester ergab 18,4 g (27 %) der Titelverbindung, Schm. 179°C.

### Beispiel 5 (all-E)-1-[4-(Tetrasol-5-yl)phenyl]-4-methyl-6-(2,6,6-trimethyl-1-cyclohexen-1-yl)-1,3,5-hexatrien

In eine Suspension von 13 g (0,2 mol) Natriumazid in 100 ml Tetrahydrofuran wurden bei 0°C vorsichtig 6,7 g (0,05 mol) Aluminiumchlorid portionsweise eingetragen. Man erhitzte 45 min unter Rückfluß, gab 3,2 g (0,01 mol) (all-E)-1-(4-Cyanophenyl)-4-methyl-6-(2,6,6-trimethyl-1-cyclohexen-1-yl)-1,3,5-hexatrien aus Beispiel 1 zu und erhitzte weitere 18 h unter Rückfluß. Das Reaktionsgemisch wurde danach auf 0,7 l Wasser gegeben, mit 200 ml Ethanol versetzt und abgesäuert. Nach längerem Verrühren bei Raumtemperatur bildete sich ein Feststoff, der abgesaugt und mit Ethanol/Heptan (1:1) gewaschen wurde. Nach Trocknen erhielt man 2,6 g (72 %) der Titelverbindung, Schmp. 195 bis 196°C.

### Beispiel 6 (all-E)-1-(4-Formylphenyl)-4-methyl-6-(2,6,6-trimethyl-1-cyclohexen-1-yl)-1,3,5-hexatrien

Zu einer Lösung von 19 g (0,06 mol) (all-E)-1-(4-Cyanophenyl)-4-methyl-6-(2,6,6-trimethyl-1-cyclohexen-1-yl)-1,3,5-hexatrien aus Beispiel 1 in einem Gemisch aus 250 ml absolutem Ether und 50 ml absolutem Tetrahydrofuran wurde unter schwacher Kühlung bei 5 bis 10°C rasch eine Lösung von 0,12 mol Diisobutylaluminiumhydrid in 100 ml Toluol zugetropft. Man ließ 2 h bei Raumtemperatur nachrühren und hydrolysierte dann vorsichtig mit Wasser und gesättigter Weinsäurelösung. Man extrahierte 7mal mit je 200 ml Ether, wusch die vereinigten Etherphasen einmal mit Wasser, trocknete (Na₂SO₄) und engte ein. Der Rückstand (20 g dunkelbraunes Öl) wurde säulenchromatographisch gereinigt (Kieselgel 60, 230 bis 400 mesh, 180 g; Heptan mit steigenden Toluolanteilen). Man erhielt so 11,1 g (58 %) der Titelverbindung, Schmp. 62 bis 63°C.

### Beispiel 7 (all-E)-1-[4-(Hydroxymethyl)phenyl]-4-methyl-6-(2,6,6-trimethyl-1-cyclohexen-1-yl)-1,3,5-hexatrien

9,6 g (0,03 mol) des im vorangegangenen Beispiel 6 beschriebenen Aldehyds wurden in 300 ml Isopropanol gelöst und bei Raumtemperatur portionsweise mit insgesamt 2,3 g (0,06 mol) Natriumborhydrid versetzt. Man ließ 2 h bei Raumtemperatur nachrühren, goßdann auf 0,6 l Wasser und extrahierte 3mal mit je 150 ml Ether. Die vereinigten Etherphasen wurden einmal mit gesättigter Natriumchloridlösung gewaschen, getrocknet (Na₂O₄) und eingeengt. Der Rückstand (8,7 g Öl) wurden mittels Säulenchromatographie gereinigt (Kieselgel 60, 230 bis 400 mesh; Heptan mit steigenden Anteilen Essigester). Das Eluat wurde nach dem Einengen durch Anreiben kristallisiert. Der Feststoff (4,4 g) wurde in 15 ml Heptan gründlich verrührt, abgesaugt und getrocknet. Man erhielt 3,0 g (30 %) der Titelverbindung; Schmp. 69 bis 70°C.

## Patentansprüche

1. Verwendung von Hexatrienderivaten der Formel I 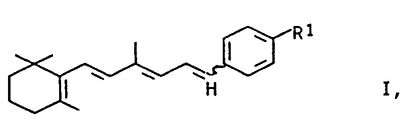 in der R¹ eine Methylgruppe, eine Nitril- oder C₂- bis C₁₀-Ketalgruppe, einen Oxazolinyl- oder Tetrazolylrest oder die Reste -CH₂OR², -CH₂NR³R⁴ oder -COR⁵ bedeutet, worin
R² ein Wasserstoffatom, eine C₁-₂₀-Alkanoyl- oder eine gegebenenfalls substituierte Benzoylgruppe,
R³ und R⁴ Wasserstoffatome, eine C₁-₄-Alkyl-, C₁-₆-Alkanoyl- oder eine gegebenenfalls substituierte Benzoylgruppe oder zusammen mit dem Stickstoffatom, an das sie gebunden sind, einen heterocyclischen Rest, und
R⁵ ein Wasserstoffatom, eine C₁-₄-Alkylgruppe, ein Halogenatom oder die Reste -OR⁶ oder -NR⁷R⁸ bedeuten, wobei
R⁶ für ein Wasserstoffatom, eine gegebenenfalls durch Hydroxylgruppen substituierte C₁-C₆-Alkylgruppe oder eine gegebenenfalls substituierte Aryl- oder gegebenenfalls im Arylteil substituierte Aralkylgruppen, und
R⁷ und R⁸ für Wasserstoffatome, gegebenenfalls durch Hydroxylgruppen substituierte C₁-C₆-Alkylgruppen oder gegebenenfalls substituierte Aryl- oder gegebenenfalls im Arylteil substituierte Aralkylgruppen oder zusammen mit dem Stickstoffatom, an das sie gebunden sind, für einen heterocyclischen Rest stehen,
sowie gegebenenfalls deren physiologisch verträglichen Salzen zur Herstellung von Mitteln gegen Vitiligo, trockene Augen sowie rheumatische und arthritische Erkrankungen.

2. Verwendung von (all-E)-1-(4-Carboxyphenyl)-4-methyl-6-(2,6,6-trimethyl-1-cyclohexen-1-yl)-1,3,5-hexatrien gemäß Anspruch 1.

3. Therapeutisches Mittel gegen Vitiligo, trockene Augen und rheumatische und arthritische Erkrankungen, das neben üblichen galenischen Hilfsmitteln eine wirksame Menge eines Hexatrienderivates der Formel I nach Anspruch 1 enthält.

4. Kosmetische Salbe, die neben üblichen Hilfsmitteln 0,001 bis 1 Gew.-% eines Hexatrienderivates der Formel I nach Anspruch 1 enthält.

5. Kosmetikum zur systemischen Anwendung, das pro Einzeldosis 0,1 bis 50 mg eines Hexatrienderivates der Formel I nach Anspruch 1 enthält.

## Claims

1. The use of hexatriene derivatives of the formula I 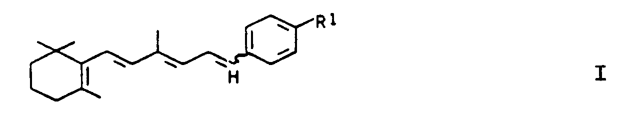 where R¹ is a methyl group, a nitrile group or C₂- to C₁₀-ketal group, an oxazolinyl or tetrazolyl radical or the radicals -CH₂OR², -CH₂NR³R⁴ or -COR⁵, where
R² is a hydrogen atom, a C₁₋₂₀-alkanoyl group or an unsubstituted or substituted benzoyl group,
R³ and R⁴ are hydrogen atoms, a C₁₋₄-alkyl group, C₁₋₆-alkanoyl group or an unsubstituted or substituted benzoyl group or, together with the nitrogen atom to which they are bonded, a heterocyclic radical, and
R⁵ is a hydrogen atom, a C₁₋₄-alkyl group, a halogen atom or the radicals -OR⁶ or -NR⁷R⁸, where
R⁶ is a hydrogen atom, a C₁-C₆-alkyl group which is unsubstituted or substituted by hydroxyl groups, or an unsubstituted or substituted aryl group or aralkyl groups which are unsubstituted or substituted in the aryl moiety, and
R⁷ and R⁸ are hydrogen atoms, C₁-C₆-alkyl groups which are unsubstituted or substituted by hydroxyl groups, or unsubstituted or substituted aryl groups or aralkyl groups which are unsubstituted or substituted in the aryl moiety, or, together with the nitrogen atom to which they are bonded, a heterocyclic radical,
as well as the physiologically tolerated salts thereof where appropriate for the preparation of compositions for vitiligo, dry eyes and rheumatic and arthritic disorders.

2. The use of (all-E)-1-(4-carboxyphenyl)-4-methyl-6-(2,6,6-trimethyl-1-cyclohexen-1-yl)-1,3,5-hexatriene as claimed in claim 1.

3. A therapeutic composition for vitiligo, dry eyes and rheumatic and arthritic disorders, which contain an effective amount of a hexatriene derivative of the formula I as claimed in claim 1, in addition to conventional pharmaceutical aids.

4. A cosmetic ointment which, in addition to conventional aids, contains from 0.001 to 1% by weight of a hexatriene derivative of the formula I as claimed in claim 1.

5. A cosmetic for systemic use which contains from 0.1 to 50 mg of hexatriene derivative of the formula I as claimed in claim 1 per single dose.

## Revendications

1. Utilisation de dérivés hexatriéniques de la formule I 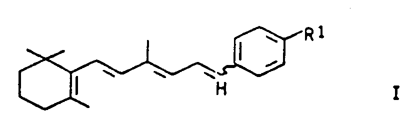 dans laquelle
R¹ représente le radical méthyle, un radical nitrile ou cétal en C₂ à C₁₀, un groupe oxazolinyle ou tétrazolyle, ou les radicaux -CH₂OR², -CH₂NR³R⁴ ou -COR⁵, où
R² représente un atome d'hydrogène, un radical alcanoyle en C₁-C₂₀, ou un radical benzoyle éventuellement substitué,
R³ et R⁴ représentent des atomes d'hydrogène, des radicaux alkyle en C₁-C₄, alcanoyle en C₁-C₆ ou des radicaux benzoyle éventuellement substitués ou forment ensemble avec l'atome d'azote auquel ils sont attachés, un radical hétérocyclique et
R⁵ représente un atome d'hydrogène, un radical alkyle en C₁-C₄, un atome d'halogène ou un groupe -OR⁶, ou -NR⁷R⁸, où
R⁶ représente un atome d'hydrogène, un radical alkyle en C₁-C₆, éventuellement substitué par des radicaux hydroxyle, ou un groupe aryle éventuellement substitué, ou un groupe aralkyle éventuellement substitué dans le radical aryle et
R⁷ et R⁸ représentent des atomes d'hydrogène, des radicaux alkyle en C₁-C₆ éventuellement substitués par des groupes hydroxyle, ou des radicaux aryle éventuellement substitués, ou des radicaux aralkyle éventuellement substitués dans les groupements aryle, ou forment ensemble avec l'atome d'azote auquel ils sont attachés, un radical hétérocyclique,
comme aussi éventuellement de leurs sels physiologiquement compatibles pour la fabrication d'agents ou compositions contre le vitiligo, les yeux secs, comme aussi contre les maladies rhumatismales et arthritiques.

2. Utilisation du (totalement-E)-1-(4-carboxyphényl)-4-méthyl-6-(2,6,6-triméthyl-1-cyclohexène-1-yl)-1,3,5-hexatriène selon la revendication 1.

3. Agent ou composition thérapeutique contre le vitiligo, les yeux secs et les maladies rhumatismales et arthritiques, contenant, outre des adjuvants galéniques usuels, une proportion active d'un dérivé hexatriénique de la formule I suivant la revendication 1.

4. Pommade cosmétique qui contient, outre des adjuvants usuels, de 0,001 à 1% en poids d'un dérivé hexatriénique de la formule I selon la revendication 1.

5. Cosmétique pour l'emploi systémique, qui contient, par dose unitaire, 0,1 à 50 mg d'un dérivé hexatriénique de la formule I selon la revendication 1.
